# EUROPÄISCHE PATENTSCHRIFT

(11) **EP 0 390 937 B1**
(45) Veröffentlichungstag und Bekanntmachung des Hinweises auf die Patenterteilung: **02.11.1994**
(21) Anmeldenummer: 89105740.8
(22) Anmeldetag: 01.04.1989
(51) Int. Cl.: A61B 17/39

(54) **Einrichtung zur Überwachung der Applikation von Neutralelektroden bei der Hochfrequenzchirurgie**
Device for the surveillance of the adherence of neutral electrodes in high-frequency surgery
Dispositif pour surveiller l'adhésion des électrodes neutres utilisées en chirurgie à haute fréquence

(43) Veröffentlichungstag der Anmeldung: 10.10.1990
(73) Patentinhaber: Erbe Elektromedizin GmbH., D-72072 Tübingen (DE)
(72) Erfinder: Farin, Günter, D-7400 Tübingen (DE); Geiselhart, Franz, D-7410 Reutlingen (DE); Klett, Johannes, D-7407 Ofterdingen (DE)
(74) Vertreter: Endlich, Fritz, Dipl.-Phys. Patentanwalt

(56) Entgegenhaltungen:
- DE-A- 3 239 640
- DE-A- 3 544 443
- FR-A- 2 516 782
- US-A- 4 657 015

## Beschreibung

Die Erfindung betrifft eine Vorrichtung zur Überwachung der Applikation von Neutralelektroden bei der Hochfrequenzchirurgie, entsprechend dem Oberbegriff des Patentanspruchs 1.

Nach DIN IEC 601 Teil 2-2 sind Neutralelektroden Elektroden von relativ großer Fläche für die Anlage an den Patientenkörper, um einen Rückflussweg für den Hochfrequenzstrom mit so niedriger Stromdichte im Körpergewebe zu bilden, daß physikalische Effekte, wie unerwünschte Verbrennungen, vermieden werden. Die Neutralelektrode sollte mit ihrer ganzen Fläche zuverlässig an den Körper des Patienten angelegt werden. Ergänzend hierzu heißt es in den Anwendungsregeln für Hochfrequenz-Chirurgiegeräte, DIN 57 753 Teil 1, es ist darauf zu achten, daß die sichere Kontaktgabe der Neutralelektrode für die Gesamtdauer der Hochfrequenz-Anwendung sichergestellt ist.

Es sind verschiedene Einrichtungen bekannt, welche die Applikation der Neutralelektrode bei der Hochfrequenzchirurgie automatisch überwachen sollen und bei unzureichender Kontaktgabe zum Körper des Patienten Warnsignale erzeugen und/oder den Hochfrequenzstrom automatisch abschalten.

Eine Einrichtung der eingangs genannten Art ist aus FR-A-2 516 782 bekannt. Bei dieser Einrichtung werden Warnsignale erzeugt und/oder der Hochfrequenzgenerator automatisch abgeschaltet, wenn die Impedanz zwischen den Kontakt flächen der zweiflächigen Neutralelektrode ermittelt wird. Dabei wird als nachteilig angesehen, daß Warnsignale erzeugt werden und/oder der Hochfrequenzgenerator auch dann abgeschaltet wird, wenn nur kleine HF-Ströme zum Schneider und/oder Koagulieren erforderlich sind, welche auch bei höheren Impedanzen keine Verbrennungsgefahr im Applikationsbereich der Neutralelektrode verursachen können. Der Anwender muß deshalb stets eine derartig große Neutralelektrode am Patienten applizieren, daß die kritische Impedanz nicht erreicht oder gar überschritten wird.

Bei einer anderen bekannten Schaltungsanordnung zur Überwachung der neutralen Elektrode (DE-A-35 44 443) wird noch als nachteilig angesehen, daß spezielle Neutralelektroden erforderlich sind, weil bei Verwendung einer zweiflächigen Neutralelektrode Verbrennungen nicht ausgeschlossen werden können. Dreiflächige Neutralelektroden sind aber nicht nur teurer als zweiflächige, sondern erfordern auch einen größeren Hardware-Aufwand.

In der deutschen Patentanmeldung PA 15 7021-24 b1 vom 02.04.1951 wird eine Sicherheitsschaltung für die Hochfrequenzchirurgie vorgeschlagen, welche dadurch gekennzeichnet ist, daß am Patienten zur Stromrückleitung wenigstens zwei neutrale Gegenelektroden angebracht werden, die mit dem Hochfrequenzgenerator über je eine getrennte Leitung in Verbindung stehen, durch welche aus einem Prüfstromkreis ein Prüfstrom geleitet wird, welcher Fehler insbesondere in der Stromrückleitung vom Patienten zum Hochfrequenzgenerator mißt und/oder anzeigt. Diese Sicherheitsschaltung ist außerdem dadurch gekennzeichnet, daß der Prüfstromkreis das Über- oder Unterschreiten zulässiger Toleranzgrenzen (d.h. zu großer oder zu kleiner Übergangswiderstände zwischen den beiden Gegenelektroden) durch ein Signal anzeigt und/oder den Hochfrequenzgenerator selbsttätig abschaltet.

In der praktischen Anwendung zeigt diese Einrichtung jedoch folgende Probleme:
Infolge mehr oder weniger großer individueller Unterschiede in der Beschaffenheit der Haut können die elektrischen Übergangswiderstände von Patient zu Patient sehr unterschiedlich sein, so daß aus dem gemessenen Übergangswiderstand nicht ausreichend sicher auf die effektive Kontaktfläche zwischen Patient und Neutralelektrode zurückgeschlossen werden kann. Bei Patienten mit trockener Haut ist der Übergangswiderstand in der Regel deutlich größer als bei Patienten mit feuchter Haut.

Außerdem gibt es heute verschiedene Typen von Neutralelektroden, z.B. konduktive, kapazitive, aus elektrisch leitfähigem Kunststoff, geliert oder trocken etc. Auch hierdurch ergeben sich verschiedene Übergangswiderstände, so daß die o.g Einrichtung nach PA 15 70 21-24 bl auf den jeweils verwendeten Typ angepaßt werden müßte.

Das Problem der individuell unterschiedlichen Beschaffenheit der Haut der Patienten soll eine aus dem deutschen Patent DE 32 39 640 C2 bekannte Überwachungsanordnung gerecht werden, die eine Anpaßeinrichtung aufweist, welche die Einrichtung zur Feststellung der oberen und der unteren Grenze des Übergangswiderstandes so steuert, daß die obere Grenze für das elektrische Signal in Abhängigkeit von dem jeweiligen Wert des elektrischen Signals eingestellt und dadurch an Änderungen der Impedanz zwischen den zwei Elektrodenelementen im Verlauf einer Behandlung angepaßt wird.

Diese Überwachungsanordnung hat jedoch den Nachteil, daß eine ausreichende Sicherheit bezüglich der Applikation der Neutralelektrode am Patienten davon abhängig ist, wie gut die Neutralelektrode vor der Messung des jeweiligen Ausgangswertes der Impedanz zwischen den zwei elektrisch voneinander isolierten Elektrodenelementen am Patienten appliziert war. Wird die Neutralelektrode in der Weise falsch am Patienten appliziert, daß eines oder beide Elektrodenelemente nur teilweise mit der Haut des Patienten in elektrisch leitfähigem Kontakt sind, so werden die obere und die untere Grenze der Impedanz von diesem fehlerhaften Anfangs-Impedanzwert aus festgelegt.

Außerdem berücksichtigt diese Einrichtung nicht die o.g. Typenvielfalt von heute auf dem Markt befindlichen Neutralelektroden, so daß diese Einrichtung ebenfalls auf den jeweils verwendeten Typ von Neutralelektrode angepaßt werden muß.

Es ist Aufgabe der Erfindung, eine Vorrichtung zu entwickeln, welche die Applikation von Neutralelektroden am Patienten in der Hochfrequenzchirurgie so überwacht, daß Warnsignale dann und nur dann erzeugt werden und/oder der Hochfrequenzgenerator des Hochfrequenz-Chirurgiegerätes dann und nur dann automatisch abgeschaltet wird, wenn das Risiko einer thermischen Schädigung der Haut des Patienten im Bereich der Kontaktflächen der Neutralelektroden vorhanden ist.

Diese Aufgabe wird erfindungsgemäß durch den Gegenstand des Patentanspruchs 1 gelöst. Vorteilhafte Weiterbildungen der Erfindung sind Gegenstand der abhängigen Ansprüche.

Die erfindungsgemäße Lösung dieser Aufgabe geht von der an sich bekannten Beobachtung aus, daß die Erwärmung elektrisch leitfähiger Stoffe durch elektrischen Strom - und dies gilt auch für biologische Gewebe - vom elektrischen Widerstand dieses Stoffes, der Stromflußdauer und insbesondere von der Intensität des elektrischen Stromes, die zum Quadrat wirkt, abhängig ist.

Um sicher zu stellen, daß auch bei großen Intensitäten und Stromflussintervallen thermische Schädigungen der Haut von Patienten im Applikationsbereich der Neutralelektroden nicht entstehen, liegt es zwar nahe, Neutralelektroden mit möglichst großen Kontaktflächen zu verwenden, um einen möglichst kleinen elektrischen Widerstand zu realisieren. Bei näherer Betrachtung stehen dem jedoch verschiedene Probleme entgegen. Relevant bezüglich der thermischen Belastung der Haut von Patienten ist nicht die auf einer Neutralelektrode zur Verfügung stehende Kontaktfläche, sondern die mit der Haut eines Patienten In Kontakt bringbare, effektive Kontaktfläche. Da die Oberfläche von Patienten nicht planflächig, sondern konkave, konvexe und/oder kegelige Formen hat, kann in der Regel nur ein Teil der zur Verfügung stehenden Kontaktfläche einer Neutralelektrode effektiv mit der Haut eines Patienten in Kontakt gebracht werden.

Außerdem ist bekannt, daß die Stromverteilung über der Kontaktfläche einer Neutralelektrode in der Regel nicht homogen ist. Je größer die Kontaktfläche einer Neutralelektrode ist, desto größer ist in der Regel auch die Inhomogenität der Stromdichte in der Kontaktfläche. Bekanntlich ist die Stromdichte in den Flächenelementen einer Neutralelektrode, welche der aktiven Elektrode am nächsten sind, am größten.

Außerdem sind große Neutralelektroden inklusive deren Verpackung teurer in der Herstellung und Entsorgung als kleinere Neutralelektroden. Letzteres gilt insbesondere für Neutralelektroden zum einmaligen Gebrauch.

Da hohe Stromintensitäten relativ selten, und wenn, dann nur relativ kurzzeitig angewendet werden, könnten in der Mehrzahl aller Operationen relativ kleinflächige Neutralelektroden angewendet werden. Um jedoch sicherzustellen, daß hohe Stromintensitäten über längere Stromflußintervalle nicht zu thermischen Schädigungen der Haut von Patienten im Applikationsbereich der Neutralelektroden führen, ist es zweckmäßig Warnsignale und/oder automatische Abschaltungen von Hochfrequenzgeneratoren nicht nur von der Höhe des Übergangswiderstandes zwischen Neutralelektrode und Patient, sondern auch von der Höhe des jeweils durch neutrale oder aktive Elektroden fließenden oder je nach Einstellung der Intensität am Hochfrequenz-Chirurgiegerät zu erwartenden Hochfrequenzstromes und der Stromflußdauer abhängig zu machen.

Eine erfindungsgemäße Vorrichtung zur Überwachung der Applikation von Neutralelektroden am Patienten bei der Hochfrequenzchirurgie besteht daher prinzipiell aus einer Einrichtung, welche den Übergangswiderstand bzw. den Übergangsleitwert zwischen Neutralelektrode und Patient in an sich bekannter Weise feststellt, einer weiteren Einrichtung, welche den per Stellglieder am Hochfrequenzgenerator eingestellten oder den während der Hochfrequenzchirurgie fließenden hochfrequenten Wechselstrom in an sich bekannter Weise feststellt und einer Verknüpfung dieser beiden Parameter in der Weise, daß einem bestimmten Übergangsleitwert ein bestimmter maximal zulässiger Hochfrequenzstrom oder einem bestimmten Hochfrequenzstrom ein bestimmter minimal zulässiger Übergangsleitwert zugeordnet wird und daß erst bei Überschreitung dieser Zuordnung Warnsignale generiert und/oder der Hochfrequenzgenerator automatisch abgeschaltet wird.
Bei der Verknüpfung des Übergangsleitwertes zwischen Neutralelektrode und Patient einerseits und dem fließenden oder zu erwartenden Hochfrequenzstrom andererseits sind verschiedene Umstände und Randbedingungen zu beachten.

Wird der Übergangswiderstand bzw. der Übergangsleitwert zwischen Neutralelektrode und Patient, wie in der Patentanmeldung PA 15 70 21-24 b1 beschrieben, mittels eines Prüfstromes ermittelt, der zwischen wenigstens zwei am Patienten angebrachten neutralen Elektroden fließt, so stellt ein derartig ermittelter Übergangsleitwert die Reihenschaltung der Übergangsleitwerte jeder neutralen Elektrode und dem Patient dar, wogegen der für die Erwärmung der Haut des Patienten relevante Übergangsleitwert nur ein Teilleitwert des so ermittelten Reihenleitwertes darstellt. Aus diesem Grunde muß der entsprechend PA 15 70 21-24 b1 ermittelte Übergangsleitwert mehr oder weniger korrigiert werden.

Da außerdem der Prüfstrom zur Ermittlung des Übergangsleitwertes nicht homogen über die Kontaktflächen der Neutralelektroden verteilt ist, ist es zweckmäßig, je nach Typ und Gestaltung der Neutralelektrode eine mehr oder weniger starke Korrektur des ermittelten Übergangsleitwertes bezüglich Typ und Gestaltung der jeweils verwendeten Neutralelektrode zu berücksichtigen.

Bei der Realisation der erfindungsgemäßen Einrichtung ist es bezüglich der Erfüllung der Aufgabe beliebig, ob die Ermittlung des relevanten Übergangsleitwertes und des relevanten Hochfrequenzstromes gleichzeitig oder zu verschiedenen Zeiten erfolgt. Da der für die Hochfrequenzchirurgie durch die aktive und/oder die neutrale Elektrode fließende Hochfrequenzstrom in der Regel viel größer ist als der für die Ermittlung des Übergangsleitwertes durch die Neutralelektroden fließende Prüfstrom, ist es jedoch mit Rücksicht auf die Störwirkung des Hochfrequenzstromes zweckmäßig, den Übergangsleitwert nicht während der Intervalle zu ermitteln, in denen der Hochfrequenzgenerator aktiviert ist, sondern während der Aktivierungspausen.

Bei einem bevorzugten Ausführungsbeispiel einer erfindungsgemäßen Einrichtung wird der Übergangsleitwert zwischen Neutralelektroden und Patient während der Aktivierungspausen des Hochfrequenzgenerators ermittelt, durch jedes Aktivierungssignal in einen Speicher übernommen und entweder mit dem am Hochfrequenz-Chirurgiegerät eingestellten oder mit dem durch Messung während der Aktivierung ermittelten Hochfrequenzstrom verknüpft und optische und/oder akustische Signale erzeugt oder der Hochfrequenzgenerator automatisch abgeschaltet, wenn oder sobald der Hochfrequenzstrom größer ist als bei dem ermittelten Übergangsleitwert maximal zulässig sein soll.

Da die Hochfrequenzströme in der Hochfrequenzchirurgie in der Regel nur sehr kurzzeitig aktiviert werden und außerdem deren Intensität während Schneide- und/oder Koagulationsvorgängen stark schwanken und auch bereits im Hochfrequenzgenerator in der Amplitude moduliert werden, ist es zweckmäßig, den Hochfrequenzstrom zu bewerten, bevor er der Verknüpfung mit dem Übergangsleitwert zugeführt wird. Bei einer Ausgestaltung der erfindungsgemäßen Einrichtung wird daher nicht der momentan fließende, sondern ein geeigneter Mittelwert des Hochfrequenzstromes für die Verknüpfung gebildet, so daß kurzzeitige Spitzenströme nicht zu Fehlalarmen oder unnötigem Abschalten des Hochfrequenzgenerators führen.

Der Vorteil der erfindungsgemäßen Einrichtung im Vergleich zu den o.g. bekannten Einrichtungen besteht darin, daß Warnsignale und/oder Abschaltungen des Hochfrequenzgenerators während der Hochfrequenzchirurgie dann und nur dann aktiv werden, wenn der Hochfrequenzstrom relativ zum jeweils vorhandenen elektrischen Übergangsleitwert zwischen Neutralelektrode und Patient zu groß ist oder zu groß werden könnte. Hierdurch können Operationen, bei denen relativ kleine Hochfrequenzströme und/oder nur kurzzeitige Einschaltungen von Hochfrequenzströmen vorhanden sind, ohne Fehlalarme weiter ausgeführt werden solange der jeweils vorhandene Übergangsleitwert zwischen Neutralelektrode und der Haut des Patienten relativ zu dem Hochfrequenzstrom nicht zu klein ist. Hierdurch ist auch die Anwendung kleinerer Neutralelektroden, die bei kleineren Hochfrequenzströmen völlig ausreichen, sicher, denn wenn, aus welchem Grunde auch immer, der Hochfrequenzstrom größer wird als für eine kleine Neutralelektrode zulässig, so erzeugt die erfindungsgemäße Einrichtung Warnsignale und/oder schaltet den Hochfrequenzgenerator ab. Kleinere Neutralelektroden sind leichter am Patienten applizierbar, kostengünstiger und leichter zu entsorgen als große Neutralelektroden.

Die erfindungsgemäße Einrichtung wird anhand schematischer Blockschaltbilder detaillierter dargestellt.
- Fig. 1: Ein schematisches Blockschaltbild eines Ausführungsbeispieles der erfindungsgemäßen Einrichtung.
- Fig. 2: Ein schematisches Blockschaltbild eines anderen Ausführungsbeispieles der erfindungsgemäßen Einrichtung.
- Fig. 3: Ein schematisches Blockschaltbild eines weiteren Ausführungsbeispieles der erfindungsgemäßen Einrichtung.

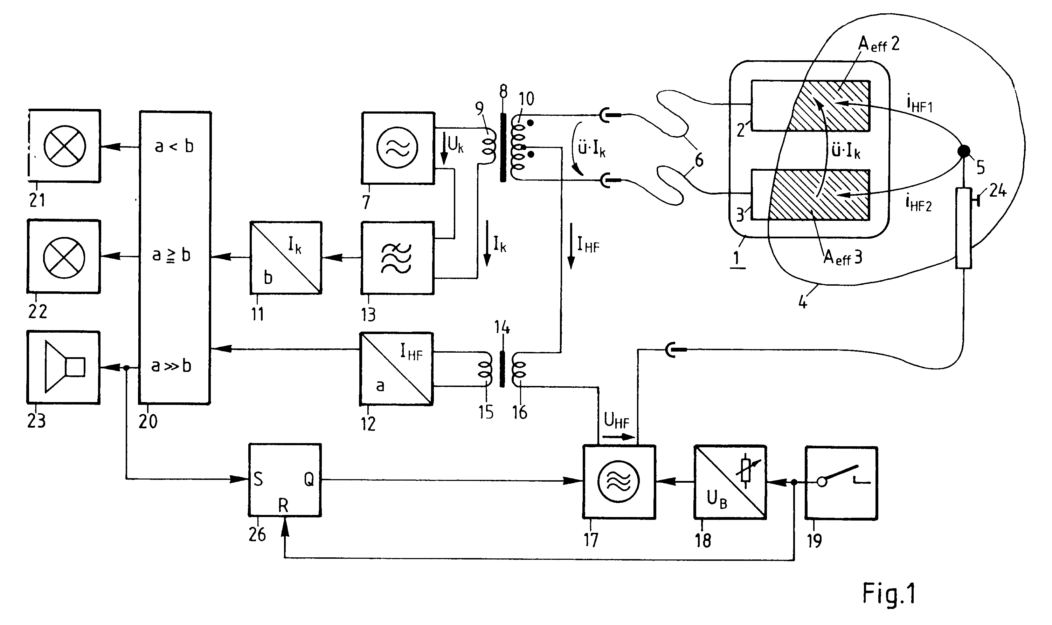

Anhand von Figur 1 wird im folgenden das Prinzip der erfindungsgemäßen Einrichtung zur Überwachung der Applikation von Neutralelektroden bei der Hochfrequenzchirurgie beschrieben.

Voraussetzung für die Überwachung der Applikation von Neutralelektroden am Patienten während der Hochfrequenzchirurgie sind entweder mindestens zwei voneinander unabhängige Neutralelektroden 2,3, die gleichzeitig am selben Patienten 4 appliziert werden bzw. eine Neutralelektrode 1 mit mindestens zwei voneinander elektrisch isolierten Kontaktflächen 2,3, die über mehradrige Kabel 6 mit dem Hochfrequenz-Chirurgiegerät verbunden werden.

In an sich bekannter Weise wird der elektrische Übergangsleitwert zwischen den Kontaktflächen 2,3 der Neutralelektrode 1 und der Haut des Patienten 4 mittels eines Kontrollstromes Iₖ ermittelt. Hierfür ist als Spannungsquelle ein mittelfrequenter Wechselstromgenerator 7 vorhanden, der über einen Transformator 8 und ein mehradriges Kabel 6 mit den Kontaktflächen 2,3 der Neutralelektrode 1 verbunden ist. Wenn die Ausgangsspannung Uₖ des Wechselspannungsgenerators 7 konstant ist, dann ist der Kontrollstrom Iₖ auf der primären Seite 9 des Transformators 8 bzw. Iₖ ^{.} ü auf der sekündären Seite 10 des Transformators 8 mit dem Übersetzungsverhältnis ü proportional dem Übergangsleitwert zwischen den Kontaktflächen 2,3 und der Haut des Patienten 4.

Wie in der Zeichnung durch zwei Punkte angedeutet ist, ist die Wicklung 10 des Transformators 8 derartig gewickelt, daß das magnetische Feld, welches durch die Teilströme I_{HF1} und I_{HF2} in der Wicklung induziert wird, bei I_{HF1} = I_{HF2} ist.

Bei Anwendung kapazitiver Neutralelektroden ist es zweckmäßig, die Frequenz des Kontrollstromes Iₖ gleich der Frequenz des hochfrequenten Stromes I_{HF} zu verwenden.

In einem Stromsensor 11 wird ein von dem Kontrollstrom Iₖ abhängiges elektrisches Signal b gebildet.

Der hochfrequente Strom I_{HF} zum Schneiden und/oder Koagulieren wird in an sich bekannter Weise in die Mitte der sekundären Wicklung 10 des Transformators 8 eingekoppelt, so daß er beide Kontaktflächen 2,3 der Neutralelektrode 1 möglichst symmetrisch erreicht. Die Höhe des hochfrequenten Stromes I_{HF} ist hauptsächlich von der Ausgangsspannung U_{HF} des Hochfrequenzgenerators 17 sowie dem elektrischen Leitwert zwischen aktiver Elektrode 5 und der Neutralelektrode 1 abhängig.

Die Höhe des hochfrequenten Stromes I_{HF} wird mittels eines Stromsensors 12, der sicherheitshalber durch einen Trenntransformator 14 vom Patienten 4 isoliert ist, ermittelt und in ein von diesem Strom abhängiges elektrisches Signal a gewandelt.

Da der elektrische Übergangsleitwert zwischen den Kontaktflächen 2,3 nicht nur von den in den Figuren 1 bis 3 schraffiert dargestellten effektiven Kontaktflächen - das meint den Teil der zur Verfügung stehenden Kontaktfläche, der mit der Haut eines Patienten leitfähig in Kontakt kommt - sondern auch von der Geometrie, dem Abstand der beiden Kontaktflächen 2,3 zueinander usw. abhängig ist, kann es vorteilhaft oder gar erforderlich sein, die Abhängigkeit des Signales b von Iₖ durch eine geeignete Funktion zu korrigieren.

Dies gilt auch bezüglich des Umstandes, daß die Höhe des Kontrollstromes Iₖ von der Reihenschaltung der Übergangsleitwerte zwischen den in den Figuren 1 bis 3 schraffiert dargestellten effektiven Kontaktflächen A_{eff2} und A_{eff3} und der Haut des jeweiligen Patienten abhängig ist, während für die thermische Belastung der Haut durch den hochfrequenten Strom I_{HF} die Parallelschaltung dieser beiden Übergangsleitwerte relevant ist. Das Verhältnis zwischen dem Übergangsleitwert, den der Kontrollstrom Iₖ vorfindet, und dem Übergangsleitwert, den der hochfrequente Strom I_{HF} vorfindet, ist abhängig von der Konstruktion der verwendeten Neutralelektrode und kann in einfacher Weise meßtechnisch für jeden Typ von Neutralelektroden ermittelt und als typischer Korrekturfaktor berücksichtigt werden.

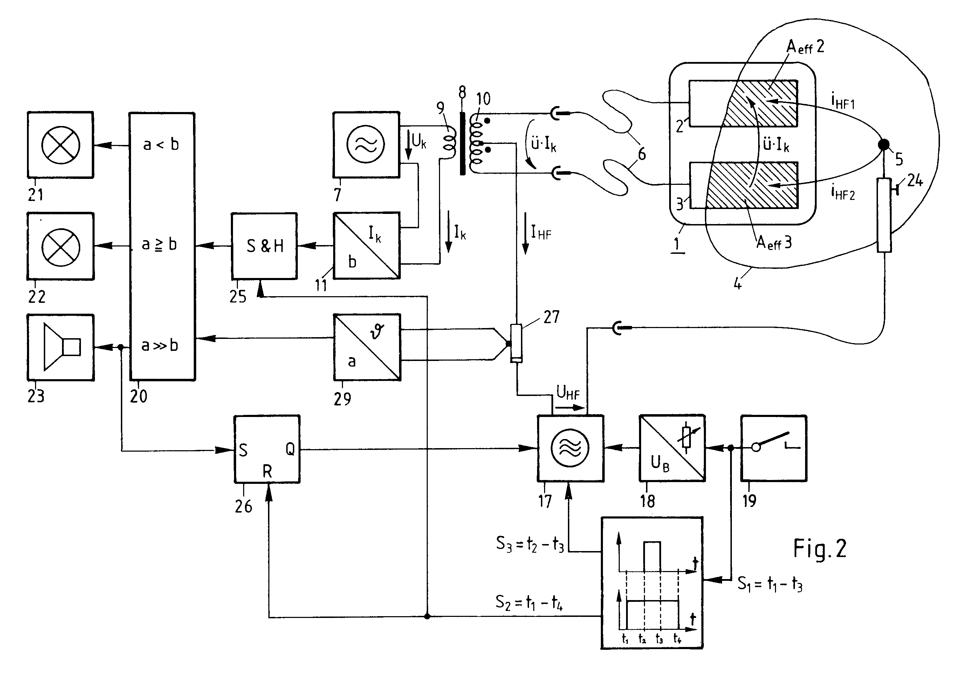

Dies gilt insbesondere auch für das Signal a in Abhängigkeit vom hochfrequenten Strom I_{HF}. Da die Hochfrequenzströme in der Hochfrequenzchirurgie in der Regel nur sehr kurzzeitig durch den Patienten fließen und außerdem deren Intensität während Schneide-und/oder Koagulationsvorgängen stark schwanken und auch bereits im Hochfrequenzgenerator mehr oder weniger stark in der Amplitude moduliert werden, und außerdem die Erwärmung des biologischen Gewebes auch von der Stromflußdauer abhängig ist, ist es zweckmäßig das Signal a in Abhängigkeit vom Effektivwert des hochfrequenten Stromes I_{HF}, z.B. wie in Fig. 2 dargestellt, mittels eines thermoelektrischen Wandlers zu ermitteln. Hierfür reicht es beispielsweise, den Strom I_{HF} durch einen reellen Widerstand 27 mit geeigneter Wärmekapazität fließen zu lassen, welche Wärmekapazität so groß ist, daß der Widerstand einerseits ausreichend warm, aber andererseits nicht so heiß wird, daß der Wandler 29 überlastet wird, während das Signal a mittels des thermoelektrischen Wandlers 29 aus der Temperatur ϑ dieses Widerstandes 27 ermittelt wird. Der Wärmewiderstand dieses Widerstandes 27 gegen das Umfeld kann konstruktiv so festgelegt werden,daß dessen Abkühlung in den Pausen etwa der Abkühlung der Haut im Kontaktbereich der Neutralelektrode entspricht, denn die an den Kontaktstellen der Neutralelektrode in der Haut des Patienten infolge des Stromes I_{HF} entstehende Wärme wird durch den Blutkreislauf und Wärmeleitung abgeleitet.
Die Erzeugung bzw. Korrektur der Signale b und/oder a in Abhängigkeit von Iₖ bzw. I_{HF} kann auch mittels digitaler Verfahren erfolgen, wofür beispielsweise Mikroprozessor-Schaltungen, mit denen in bekannter Weise beliebige Abhängigkeiten des Signals b von Iₖ und/oder a von I_{HF} realisierbar sind, Verwendung finden können.

Wenn ein relativ großer hochfrequenter Strom I_{HF} und ein relativ kleiner Kontrollstrom Iₖ gleichzeitig durch die sekundäre Wicklung 10 des Transformators 8 fließen, kann die Auswertung des Kontrollstromes Iₖ gestört werden, wenn der Strom I_{HF} sich nicht gleichmäßig auf beide Kontaktflächen 2,3 der Neutralelektrode 1 verteilt, d.h. wenn i_{HF1} kleiner oder größer als I_{HF2} ist. Deswegen ist, wie in Figur 1 dargestellt, ein Filter 13 zweckmäßig oder gar erforderlich, welches eine Störung der Auswertung von Iₖ durch eine Differenz der hochfrequenten Teilströme i_{HF1} und i_{HF2} unterdrückt, indem es Ströme mit der Frequenz des Hochfrequenzgenerators 17 sperrt.

Da hochfrequenter Strom I_{HF} einerseits nur kurzzeitig während Schneide- und/oder Koagulationsvorgängen vorhanden ist, und andererseits das Risiko, daß die Applikation einer Neutralelektrode sich während dieser kurzzeitigen Vorgänge wesentlich verschlechtert, vernachlässigbar klein ist, kann der Wert des Signals b, der vor jedem Aktivieren des Hochfrequenzgenerators 17 vorhanden ist, wie in Figur 2 dargestellt, während einer jeden Aktivierungsperiode t₂ - t₃ des Hochfrequenzgenerators 17 durch eine Sample and Hold-Schaltung 25 festgehalten werden. Hierbei ist es zweckmäßig, die Aktivierung des Hochfrequenzgenerators 17 und die Sample and Hold-Schaltung 25 zeitlich so zu steuern, daß die Aktivierungsintervalle t₂ - t₃ stets innerhalb der Hold-Intervalle t₁ - t₄ der Sample and Hold-Schaltung liegen.

Die elektrischen Signale a und b werden einem Komparator 20 zugeführt, welcher diese beiden Signale miteinander vergleicht und je nach deren Verhältnis zueinander Steuersignale erzeugt, deren Anwendung je nach Bedarf definiert werden kann. So kann beispielsweise bei a < b ein grünes optisches Signal 21, bei a ≧ b ein rotes optisches Signal 22 und/oder bei a » b ein akustisches Signal 23 aktiviert und/oder gleichzeitig der Hochfrequenzgenerator 17 deaktiviert werden, wobei der Hochfrequenzgenerator 17 solange deaktiviert bleibt, bis der Aktivierungsschalter 19 erneut geschlossen wird und hierdurch ein RS-Flip-Flop 25 zurücksetzt. Statt des Hochfrequenzgenerators 17 könnte alternativ auch das Netzteil 18 deaktiviert werden.

Der Aktivierungsschalter 19 kann ein per Fingertaste 24 an der aktiven Elektrode 5 betätigbarer Fingerschalter, ein per Pedal betätigbarer Fußschalter oder ein automatisch betätigter Relais-Kontakt sein.

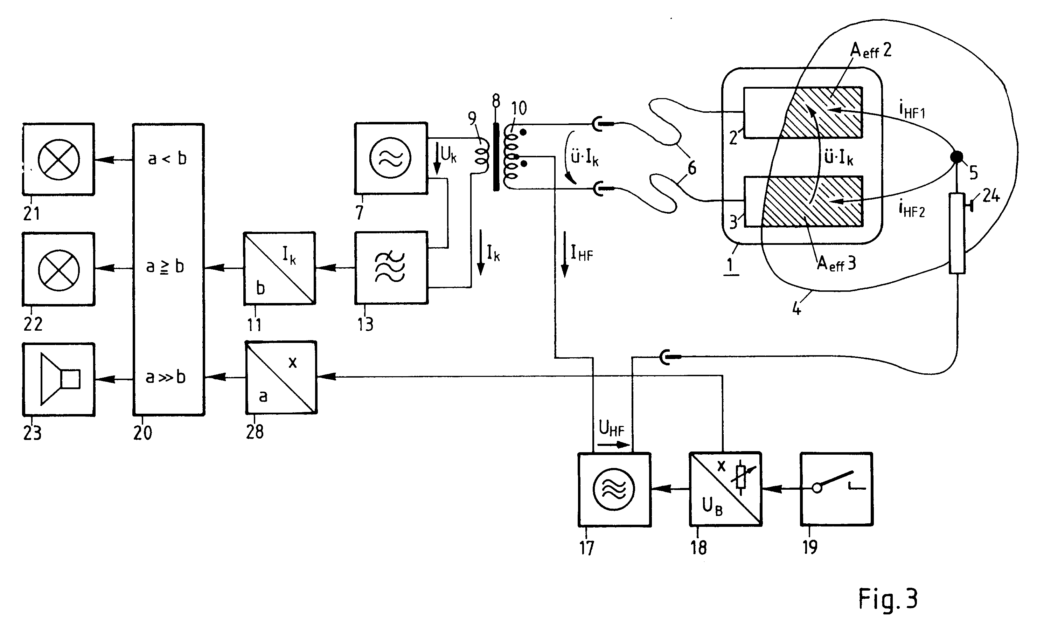

Eine andere Lösung der Aufgabe wird anhand der Figur 3 beschrieben. Diese Lösung unterscheidet sich von den in den Figuren 1 und 2 dargestellten Lösungen dadurch, daß das Signal a nicht vom tatsächlich durch den Patienten 4 fließenden hochfrequenten Strom I_{HF}, sondern von dem am Hochfrequenz-Chirurgiegerät, beispielsweise am Netzteil 18 eingestellten, für die Erwärmung des biologischen Gewebes relevanten Parameter x abgeleitet wird. Hierbei kann x die Ausgangsspannung U_{HF} des Hochfrequenzgenerators 17, der Ausgangsstrom I_{HF} oder die Ausgangsleistung P_{HF} des Hochfrequenzgenerators 17 sein.

Die Abhängigkeit des Signales a vom Parameter x kann in einer Bewertungsschaltung 28, welche beispielsweise mittels Mikroprozessor realisierbar ist, wie bei den Ausführungsbeispielen der Figuren 1 und 2 beschrieben, definiert und/oder korrigiert werden.

Alle anderen Eigenschaften dieses Ausführungsbeispieles entsprechen den in den Figuren 1 und 2 dargestellten und beschriebenen Ausführungsbeispielen.

## Patentansprüche

1. Vorrichtung zur Überwachung der Applikation von Neutralelektroden bei der Hochfrequenzchirurgie mit einer aktiven Elektrode (5), wobei die Kontaktflächen (2,3) von mindestens zwei voneinander unabhängigen Neutralelektroden oder eine Neutralelektrode (1) mit mindestens zwei voneinander elektrisch isolierten Kontaktflächen (2,3) an einem Patienten (4) appliziert werden, mit
a) einer ersten Einrichtung (7,8,9,10,11,13), welche ein elektrisches Signal b in Abhängigkeit vom elektrischen Übergangsleitwert zwischen den Kontaktflächen (2,3) liefert,
**dadurch gekennzeichnet,** daß
b) eine zweite Einrichtung (12,14,15,16 oder 27,29) vorgesehen ist, welche ein elektrisches Signal a in Abhängigkeit vom Hochfrequenzstrom (I_{HF}) liefert, der durch die voneinander unabhängigen Neutralelektroden bzw. durch die Neutralelektrode (1) oder durch die aktive Elektrode (5) fließt, oder eine Bewertungsschaltung (28) vorgesehen ist, welche das elektrische Signal a in Abhängigkeit von einem am Netzteil (18) des Hochfrequenzchirurgiegerätes eingestellten elektrischen Signal X liefert, welches Signal X dem Sollwert der Betriebsspannung (U_{B}), der Ausgangsspannung (U_{HF}), des Hochfrequenzstromes (I_{HF}) oder der Ausgangsleistung (P_{HF}) des Hochfrequenzgenerators entspricht, und daß
c) ein Komparator (20) vorgesehen ist, welchem die Signale a und b zugeführt werden und der jeweils mindestens ein Steuersignal liefert, je nachdem a < b oder a ≧ b und/oder a » b ist, wodurch eine optische und/oder akustische Signaleinrichtung (21,22,23) aktiviert und/oder der Hochfrequenzstrom (IHF) abgeschaltet wird.

2. Vorrichtung nach Anspruch 1, **dadurch gekennzeichnet,** daß das elektrische Signal a in Abhängigkeit vom Effektivwert des Hochfrequenzstromes (I_{HF}) mittels eines thermoelektrischen Wandlers (29) ermittelt wird, indem der Hochfrequenzstrom (I_{HF}) durch einen reellen Widerstand (27) mit geeigneter Wärmekapazität und definiertem Wärmewiderstand gegen das Umfeld geleitet wird und das Signal a aus der Temperatur (ϑ) dieses Widerstandes (27) ermittelt wird.

3. Vorrichtung nach Anspruch 1 oder 2, **dadurch gekennzeichnet,** daß das Signal b einer Sample & Hold-Schaltung (25) zugeführt wird, welche den Wert des Signals b während Pausenintervallen, wenn der Hochfrequenzgenerator (17) nicht aktiviert ist, aus der ersten Einrichtung (7,8,9,10,11) zur Lieferung des Signals b kontinuierlich übernimmt und während Aktivierungsintervallen t₂-t₃ denjenigen Wert des Signals b speichert und an den Komparator (20) weiterleitet, welcher vor dem Zeitpunkt t₂vorhanden war.

4. Vorrichtung nach Anspruch 1, **dadurch gekennzeichnet,** daß ein erster optischer Signalgeber (21) eingeschaltet wird, wenn a < b ist.

5. Vorrichtung nach Anspruch 1, **dadurch gekennzeichnet,** daß ein zweiter optischer Signalgeber (22) eingeschaltet wird, wenn a ≧ b ist.

6. Vorrichtung nach Anspruch 1, **dadurch gekennzeichnet,** daß ein akustischer Signalgeber (23) aktiviert und/oder der Hochfrequenzgenerator (17) automatisch abgeschaltet wird, wenn a ≧ b oder a » b ist.

7. Vorrichtung nach Anspruch 1, **dadurch gekennzeichnet,** daß die Abhängigkeit des elektrischen Signals b vom Übergangsleitwert oder von einem Kontrollstrom (Iₖ) mittels eines Mikroprozessors und geeigneter Software entsprechend den relevanten Randbedingungen korrigiert wird.

8. Vorrichtung nach Anspruch 1 oder 2, **dadurch gekennzeichnet,** daß die Abhängigkeit des elektrischen Signals a vom Hochfrequenzstrom (I_{HF}) mittels eines Mikroprozessors und geeigneter Software entsprechend den relevanten Randbedingungen korrigiert wird.

## Claims

1. Apparatus for monitoring the application of neutral electrodes in high frequency surgery with an active electrode (5), the contact faces (2, 3) of at least two independent neutral electrodes or one neutral electrode (1) having at least two contact faces (2, 3) which are electrically insulated against one another are applied to a patient (4) with
a) a first device (7, 8, 9, 10, 11, 13), which supplies an electrical signal b as a function of the electric transition conductance between the contact faces (2, 3),
characterized in that
b) a second device (12, 14, 15, 16 or 27, 29) is provided, which supplies an electrical signal a as a function of high frequency current (I_{HF}), which flows through the independent neutral electrodes or through the neutral electrode (1) or through the active electrode (5), or an evaluating circuit (28) is provided, which supplies the electrical signal a as a function of an electrical signal X set on a mains part (18) of the high frequency surgery equipment, said signal X corresponding to the desired value of the operating voltage (U_{B}), the output voltage (U_{HF}), the high frequency current (I_{HF}) or the output power (P_{HF}) of the high frequency generator and that
c) a comparator (20) is provided to which are supplied the signals a and b and which in each case supplies at least one control signal, as a function of whether a < b or a ≧ b and/or a » b, so that an optical and/or acoustic signal device (21, 22, 23) is activated and/or the high frequency current (I_{HF}) is switched off.

2. Apparatus according to claim 1, characterized in that the electrical signal a is determined as a function of the r.m.s. value of the high frequency current (I_{HF}) by means of a thermoelectric converter (29), in that the high frequency current (I_{HF}) is passed through a real resistor (27) with a suitable thermal capacity and a defined thermal resistance with respect to the surrounding field and the signal a is determined from the temperature (ϑ) of said resistance (27).

3. Apparatus according to claim 1 or 2, characterized in that the signal b is supplied to a sample and hold circuit (25), which continuously takes over the value of the signal b during intervals, when the high frequency generator (17) is not activated, from the first device (7, 8, 9, 10, 11) for the supply of the signal b and during the activation intervals t₂-t₃ stores said value of the signal b and passes it to the comparator (20), which was present before the time t₂.

4. Apparatus according to claim 1, characterized in that a first optical signal generator (21) is switched on if a < b.

5. Apparatus according to claim 1, characterized in that a second optical signal generator (22) is switched on if a ≧ b.

6. Apparatus according to claim 1, characterized in that an acoustic signal generator (23) is activated and/or the high frequency generator (17) is automatically switched off if a ≧ b or a » b.

7. Apparatus according to claim 1, characterized in that the dependence of the electric signal b on the transition conductance or a control current (Iₖ) is corrected by means of a microprocessor and suitable software in accordance with the relevant boundary conditions.

8. Apparatus according to claim 1 or 2, characterized in that the dependence of the electric signal a on the high frequency current (I_{HF}) is corrected by means of a microprocessor and suitable software in accordance with the relevant boundary conditions.

## Revendications

1. Dispositif de surveillance de l'application d'électrodes neutres dans la chirurgie à haute fréquence, avec une électrode active (5), dans lequel les surfaces de contact (2, 3) d'au moins deux électrodes neutres indépendantes l'une de l'autre ou une électrode neutre (1) pourvue d'au moins deux surfaces de contact (2, 3) électriquement isolées l'une de l'autre sont appliquées sur un patient (4), avec
a) un premier équipement (7, 8, 9, 10, 11, 13) qui émet un signal électrique b en fonction de la conductance de contact entre les surfaces de contact (2, 3),
caractérisé
b) en ce qu'il est prévu un second équipement (12, 14, 15, 16 ou 27, 29) qui émet un signal électrique a en fonction du courant haute fréquence (I_{HF}) qui passe dans les électrodes neutres indépendantes l'une de l'autre ou dans l'électrode neutre (1) ou dans l'électrode active (5), ou bien qu'il est prévu un circuit d'évaluation (28) qui émet le signal électrique a en fonction d'un signal électrique X prescrit dans le bloc d'alimentation secteur (18) de l'appareil de chirurgie à haute fréquence, lequel signal X correspond à la valeur prescrite de la tension de service (U_{B}), de la tension de sortie (U_{HF}),du courant haute fréquence (I_{HF}) ou de la puissance de sortie (P_{HF}) du générateur haute fréquence, et
c) en ce qu'il est prévu un comparateur (20) auquel sont amenés les signaux a et b et qui émet au moins un signal de commande selon que l'on a chaque fois a » b ou a ≧ b et /ou a»b, ce par quoi un équipement (21, 22, 23) de signalisation optique et/ou acoustique est activé et/ou le courant haute fréquence (I_{HF}) est mis hors circuit.

2. Dispositif selon la revendication 1, caractérisé en ce que le signal électrique a est déterminé en fonction de la valeur efficace du courant haute fréquence (I_{HF}) au moyen d'un convertisseur thermoélectrique (29), en faisant passer le courant haute fréquence (I_{HF}) dans une résistance réelle (27) de capacité calorifique appropriée et de résistance thermique définie à l'égard de l'environnement et en ce que le signal a est déterminé d'après la température (ϑ) de cette résistance (27).

3. Dispositif selon la revendication 1 ou 2, caractérisé en ce que le signal b est amené à un circuit d'échantillonnage (25) qui relève en continu, sur le premier équipement (7, 8, 9, 10, 11) de fourniture du signal b, la valeur du signal b pendant les intervalles de repos, lorsque le générateur haute fréquence (17) n'est pas activé, et mémorise, pendant les intervalles d'activation t₂ - t₃, et transmet au comparateur (20), la valeur du signal b qui existait avant l'instant t₂.

4. Dispositif selon la revendication 1, caractérisé en ce qu'un premier émetteur (21) de signal optique est mis en circuit lorsque a<b.

5. Dispositif selon la revendication 1, caractérisé en ce qu'un second émetteur (22) de signal optique est mis en circuit lorsque a ≧ b.

6. Dispositif selon la revendication 1, caractérisé en ce qu'un émetteur (23) de signal acoustique est activé et/ou que le générateur haute fréquence (17) est automatiquement mis hors circuit lorsque a ≧ b ou a » b.

7. Dispositif selon la revendication 1, caractérisé en ce que la dépendance du signal électrique b à l'égard de la conductance de contact ou d'un courant de contrôle (Iₖ) est corrigée, au moyen d'un microprocesseur et d'un logiciel approprié, en fonction des conditions aux limites pertinentes.

8. Dispositif selon la revendication 1 ou 2, caractérisé en ce que la dépendance du signal électrique a à l'égard du courant haute fréquence (I_{HF}) est corrigée, au moyen d'un microprocesseur et d'un logiciel approprié, en fonction des conditions limites pertinentes.
